# EUROPEAN PATENT SPECIFICATION

(11) **EP 3 324 182 B1**
(45) Date of publication and mention of the grant of the patent: **26.02.2020**
(21) Application number: 16002477.4
(22) Date of filing: 22.11.2016
(51) Int. Cl.: G01N 33/24, G01N 23/204

(54) **MOISTURE MEASUREMENT APPARATUS**
FEUCHTIGKEITSMESSVORRICHTUNG
APPAREIL DE MESURE D'HUMIDITÉ

(43) Date of publication of application: 23.05.2018
(73) Proprietor: Universität Heidelberg, 69117 Heidelberg (DE)
(72) Inventor: Köhli, Markus, 77966 Kappel-Grafenhausen (DE); Schmidt, Ulrich, 69221 Dossenheim (DE)
(74) Representative: Müller-Boré & Partner Patentanwälte PartG mbB

(56) References cited:
- Martin Schrön ET AL: "Soil Moisture Estimation Across Scales with Mobile Sensors for Cosmic-Ray Neutrons from the Ground and Air", Geophysical Research Abstracts, 17 April 2016 (2016-04-17), XP055359788, Retrieved from the Internet: URL:http://meetingorganizer.copernicus.org /EGU2016/EGU2016-16720-3.pdf [retrieved on 2017-03-29]
- VICTOR V. KLEMAS: "Coastal and Environmental Remote Sensing from Unmanned Aerial Vehicles: An Overview", JOURNAL OF COASTAL RESEARCH, vol. 315, 2 September 2015 (2015-09-02), pages 1260-1267, XP055359689, ISSN: 0749-0208, DOI: 10.2112/JCOASTRES-D-15-00005.1
- HENSKE M ET AL: "TheB based Jalousie neutron detector An alternative forHe filled position sensitive counter tubes", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 686, 21 May 2012 (2012-05-21), pages 151-155, XP028400167, ISSN: 0168-9002, DOI: 10.1016/J.NIMA.2012.05.075 [retrieved on 2012-06-02]

## Description

The present invention relates to a moisture measurement apparatus, particularly a soil moisture measurement apparatus and a related method based on cosmic ray neutron probing (CRNP) and particularly based on cosmic ray induced atmospheric neutron probing. In this respect, it is also simply referred to neutrons and neutron probing or neutron sensing in the present application.

In recent years, ecological disasters have increased tremendously in numbers and damage. Flooding and possible landslip related to flooding are some of the phenomena causing enormous damage and endangering human lives in multiple regions and countries of the earth. Predicting the probability and an acute risk of flooding is therefore essential for limiting damage, evacuation and saving lives. However, a reliable prediction is object of ongoing research and still remains challenging.

One important parameter for predicting phenomena related to flooding is the moisture/water content of soil and the degree of soil water saturation. Thus, determining soil moisture content plays an essential role in disaster predictions, environmental monitoring, and hydrological applications.

Another field which requires the reliable determination of soil moisture content may be farming, particularly sustainable and precision farming. It is important for a farmer to know exact amounts of moisture contained in the soil for determining the correct/optimal amount of irrigation, for example. This may help to achieve optimal growing results, to avoid salting of the soil, to avoid flooding and to safe water which is particularly important in dry areas.

Martin Schrön et al. ("Soil Moisture Estimation Across Scales with Mobile Sensors for Cosmic-Ray Neutrons from the Ground and Air", Geophysical Research Abstracts, 17. April 2016) discloses a method for soil moisture estimation based on cosmic-ray neutron measurements which are performed using a gyrocopter.

Victor V. Klemas ("Coastal and Environmental Remote Sensing from Unmanned Aerial Vehicles: An Overview", Journal of Coastal Research, vol. 31, issue 5, 1. September 2015, ISSN: 0749-0208, DOI: 10.2112/JCOASTRES-D-15-00005.1") discloses the use of unmanned aerial vehicles, such as unmanned aircrafts, helicopters and balloons for example for land use mapping and wetlands mapping.

Henske M. et al. ("The 10B based Jalousie neutron detector - An alternative for 3He filled position sensitive counter tubes", Nuclear Instruments & Methods in Physics Research, Section A: Accelerators, Spectrometers, Detectors, and associated Equipment, Elsevier BV* North-Holland, NL, vol. 686, 21. May 2012, pages 151-155, ISSN: 0168-9002, DOI: 10.1016/J.NIMA.2012.05.075) discloses a ¹⁰B based Jalousie neutron detector as an alternative for ³He filled position sensitive counter tubes.

In this respect, it is preferable to provide an eco-friendly, simple and reliable technique for non-invasively measuring the soil moisture of large land areas. Such areas may possibly be located in impassable and rough terrain. In view of these factors, multiple techniques have been developed for measuring the water/moisture content in soil.

To date, soil moisture content has been determined by means of local contact-based (invasive) techniques. One such technique is based on weighing a portion of soil including soil moisture content, weighing the same portion of soil after heating and evaporating the moisture content. The moisture content is then determined by subtracting the measured weights from each other. Alternatively, soil moisture content may be determined by measuring the electrical resistance while applying an electrical field to a portion of soil. Other methods for detecting soil moisture content include time domain - or frequency domain reflectometry, which requires injecting a wire into a portion of soil. Further, a Troxler detection head has been used to introduce neutrons locally into a portion of soil and to measure thermalization. These well-established methods have been known and applied for decades. They are contact-based and allow for distinct local probing. Further, most of these methods - being invasive - require insertion into the soil or permanent positioning below the soil.

The soil water content may also be estimated by means of satellite based systems detecting radio or optical signals. This allows to roughly scan large areas in view of hydrologic applications, as the altitude of satellites is extremely large compared to other systems.

A technique which has recently been developed and is still object of ongoing research is based on a signal to noise ratio (SNR) recorded by high precision GPS receivers, wherein such receivers are already in place for geodetic applications. Some of the noise in a GPS signal comes from reflections of the GPS signal off of the ground. These reflected signals come in and out of phase with the direct signal as a GPS satellite rises, and as a result, the recorded SNR data rise and fall over this time period, creating a sine curve. The shape of this SNR sine curve changes depending on the amount of water in the soil.

An alternative non-invasive technique for measuring soil water content in the range of several square kilometers, related to weather observation, is based on optical spectral detection of the Earth's surface with satellites, known as remote sensing. Areas under clouds can hardly be probed using satellite based soil moisture content detection. The first centimeter of the soil may be probed with this method. Vertical inhomogeneity of soil moisture content further requires careful analysis of measured data using precise soil models.

Cosmic ray neutron sensing or cosmic ray neutron probing (CRNP) - another non-invasive contactless technique to identify soil moisture content - has recently been introduced for the first time (in 2008). This method is based on the detection of neutron density related to such neutrons which have been reflected/scattered back from the soil. These neutrons are particularly sensitive to and indicative of soil moisture content. Such neutrons are cosmic ray induced atmospheric neutrons. The origin of these neutrons is discussed in more detail further below.

To date, thermal neutron detectors have been applied for this technique (mainly produced by the company Hydroinnova). Such detectors can be installed at fixed positions or transported in a vehicle to provide a degree of mobility. Other means for providing a high degree of mobility have been reported, such as gyrocopters or microlights. Therefore, the benefits of transporting the detector at a high altitude during detection, such as probing a large area, can be exploited.

However, it still remains challenging to provide a technology which allows for reliably scanning and/or imaging soil moisture content in large areas at realistic costs.

According to an aspect, it is a problem to determine the soil moisture content of large areas with an improved degree of reliability. The problem is solved by an apparatus and a method having the features defined in the independent claims. Preferred embodiments are covered by the dependent claims.

The present invention relates to an apparatus and a respective method for probing moisture content and more particularly soil moisture content by means of detecting neutrons. The spatial range which may be covered by the invention (including the size of the observable area of typically multiple hectares and the special resolution) lies in a regime which has so far been challenging to be covered by means of conventional probing techniques. In particular, the size of the observable area is larger than for typical conventional local detecting systems. On the other hand, the spatial resolution achieved by the present disclosure is higher than for typical conventional satellite based detection systems.

Therefore, the apparatus and method according to the present disclosure may be applied for passive mobile large scale hydrological measurements, particularly for flood control and prevention and for farming and particularly for precision farming. The field of the present disclosure is however not limited to said application and may be extended to other meteorological and/or biological fields such as weather observation, observation of cloud and/or fog/mist development and/or water accumulation and/or vegetal cover and/or snow cover as well as the condition of snow fields.

According to the present invention a moisture measurement apparatus is provided for detecting moisture content, particularly soil moisture content. The apparatus is defined in claim 1.

Airships and related "lighter-than-air technologies" have long been known to provide multiple advantages, such as being eco-friendly and cheap due to low energy consumption, low noise production, ability to transport heavy loads from point to point, ability to move far distances while being flexible in reaching various altitudes and being mobile over impassable and rough terrain. Further, as opposed to aerodynes, such as planes, helicopters, gyrocopters or drones, airships are able to move/float over land at very low pace up to standing still with respect to the geological coordinate. Further, airships are not required to be started from a starting-/landing strip. The restrictions regarding the starting and/or landing positions of airships are low, in general. Further, depending on the carrier gas used in the airship, the risk for accidents can be held very low.

For the above reasons, it is also preferable to provide an airship for neutron sensing. All said advantages related to airships can thus be exploited in conjunction with determining soil moisture content.

It is particularly beneficial to provide an airship for neutron sensing as it may float at altitudes which allow probing large surface areas and large spatial coverage. Such areas may possibly be located in impassable and rough terrain. This is of particular interest as the risk of landslip due to high soil water content is usually high at mountains and in mountainous countryside which is often rough and hard to approach. It is beneficial in view of versatility to use a technique for scanning large areas (i.e. several hectares and more) rather than using a technique which allows for local measurements for evaluating and observing the hydrological condition as well as the degree of saturation of soil water in a vast landscape. For example, an observation area of about one to ten hectares may be probed quickly and precisely using the apparatus according to the present invention. Moreover, a scanned observation area may also be larger. Alternatively, the observation area to be scanned and probed may also be smaller, particularly in the case when the airship is fixed and/or tethered to a fixed position over the ground. As opposed to conventional local, static detection systems, the present invention allows scanning larger areas.

On the other hand, as opposed to conventional satellite based systems, the present invention provides a more reliable detection with a higher spatial resolution. Further, mobile neutron sensing according to the present disclosure does not require a large-scale network of sensors at fixed positions, hence being less extensive and expensive in view of installation and maintenance. Mobile neutron sensing according to the present disclosure also allows a user to apply the technique wherever needed acutely. At the same time, the present disclosure also allows to take into account soil moisture content present in several decimeters below the earth's surface, thus providing a more reliable conclusion about the soil's saturation with water.

As an airship can float at very low floating pace, multiple measurements may be recorded for one area as the airship moves over small distances, thus being considered quasi-static in a rough approximation. The temporal window for reliably detecting soil moisture content of a particular area, being determined by the floating pace, is therefore improved. This gives rise to improved accuracy, improved spatial resolution and as a result a higher degree of reliability. Further, neutron sensing is a non-invasive, sensitive as well as reliable technique for probing soil moisture content. Therefore, the apparatus and related method according to the present invention can improve efficiency and reliability when implemented in an early-warning system.

The at least one airship is at least partially filled with a carrier gas when floating. In a non-filled state, i.e. when the gas-filled volume of the airship is compressed and not filled with a gas, the airship may easily be transported as the volume is reduced down to small sizes. When the volume is at least partially filled with a carrier gas, the airship can adopt at least in parts its full size. In this case, the airship uses the buoyancy force for floating as the carrier gas is lighter than air. As opposed to other aircrafts, such as helicopters or gyrocopters, a gas-filled airship can exploit the buoyancy force at very high altitudes.

The carrier gas at least partially serves as the at least one moderator medium. It is according to the invention to fill the volume of the airship with a gas which is used at least in parts as a carrier medium and as a moderator medium (i.e. for slowing down the neutrons before detection) at the same time. In the process of moderation, fast neutrons are slowed down to kinetic energies at which they can effectively be detected by the neutron detector. Often, the neutron detectors which are used for neutron sensing comprise a solid state moderator medium, such as polyethylene. A polyethylene moderator medium can weigh as much as 8 to 30 kg. Therefore the additional weight may be reduced or completely avoided if the carrier gas has a moderating function. If a small aircraft is to be used for neutron sensing, such weights to be carried would limit or render the measurement impossible. Favorably, the airship may simply carry the thermal neutron detector's weight and its own weight, as the carrier gas is lighter than air and does have the functions of carrying the apparatus and moderating the neutrons at the same time. Therefore, an efficient apparatus can be provided having a large detection volume, floating slowly at low altitudes and simply carrying the weight of the sensor if no additional equipment is to be carried. Energy consumption and costs may therefore be reduced.

According to an embodiment, the carrier gas is at least partially one out of hydrogen, helium, heated air, methane, ammonia, neon, acetylene, diborane, carbon monoxide, nitrogen and ethene or a mixture thereof. A carrier gas which contains hydrogen atoms, such as hydrogen or methane, is efficient in moderating neutrons. Making use of such compounds is therefore beneficial, as they serve efficiently as the carrier gas and the moderator medium at the same time. Further, it is beneficial in some cases to use for example hydrogen gas instead of helium gas as a carrier gas, as hydrogen is more efficient in its buoyancy properties and less expensive. For safety reasons it may however also be favorable to provide helium gas instead of hydrogen gas, as it is inert and not explosive. Excluding for example mixtures of gases such as oxyhydrogen gases which would cause combustion, it might also be beneficial to provide other said gases or mixtures of said gases in order to tune moderator and/or carrying features. Separate gas chambers may therefore be provided to isolate different gases in the volume of the airship.

According to the invention, the at least one neutron detector is at least partially surrounded by the carrier gas. If the neutron detector is at least partially surrounded by the carrier gas, the neutrons can be slowed down in the vicinity of the detector, as the carrier gas is also used as the moderator medium. The gas phase which at least partially surrounds the detector may then substitute a moderator shell such as a polyethylene case or cover.

According to an embodiment, the at least one neutron detector comprises at least one gas proportional detector, particularly a boron neutron converter and more particularly a Jalousie detector and/or a scintillation neutron detector and/or a semiconductor neutron detector.

As opposed to ³He neutron detectors, boron neutron converters (¹⁰B solid state detector), such as Jalousie detectors, are far less expensive. Therefore, a probing interval can be substantially extended to long duration. Large areas of landscape may therefore be probed with boron neutron converters. On the contrary, ³He is expensive and as a result only small amounts of ³He may be provided in a detector such that only small areas of landscapes can be probed. With boron detectors however, soil moisture content of a larger area and at a higher spatial resolution can be achieved during a typical probing interval of one day. It can also be beneficial to provide multiple detectors in view of expanding the probing intervals or further improving reliability. It cannot be excluded that, in some cases, it is beneficial to provide detectors based on other technologies (in addition or as an alternative to boron neutron converters), particularly if such detectors are cheaper and/or more reliable and/or having a longer life time.

According to an embodiment, the apparatus further comprises at least one:
- second measurement device configured to detect at least one out of humidity, pressure, background radiation, background neutron density, floating altitude, temperature, time lapse, weight of a carried object, carrier gas volume and/or carrier gas pressure, geomorphology and/or natural vegetal cover within the observation area of the earth's surface; and/or
- camera configured for imaging the geographic landscape.

Recording other measures which do not involve neutron energy (as a derivative), such as the above mentioned ones, is favorable as conditions can be recorded and measures which may help deriving the soil moisture content from the neutron density and the energy distribution can be detected. Such measures, as for example barometric pressure or temperature may be useful for the calibration of the neutron detector. However, such values can also be recorded independently from the neutron detection from additional meteorological observations. A manifold of parameters can be detected and fed into a model for weather prediction, severe weather prediction and/or ecological disasters prediction related to weather phenomena.

According to an embodiment, conditions and/or features and/or functions of the apparatus, such as float velocity, position, floating altitude, carrier gas pressure and/or detection of measures are controlled dynamically and/or in a predetermined manner by means of at least one control element out of a remote control, a computer, a smartphone, a satellite, or a base station and/or data may be exchanged between the control element and the apparatus.

An operator may dynamically derive a condition of the soil and/or weather from the detected data when the data is exchanged between (elements of) the apparatus and control elements on the ground, such as a remote control and/or a computer/server. In other words, it may be advantageous to transfer data between detection elements and computers and/or servers, e.g. via mobile Internet, for instantaneous storage and/or analysis of the exchanged data. It is also possible that communication takes place between several control elements and the airship via a receiver or transceiver at the apparatus or airship. For example, a GPS signal of satellites may be received by a receiver in the airship, the receiver may be connected to a computer either in/at the airship or on the ground such that the computer can derive the exact position and floating velocity. Based on such data, an operator can control the floating trajectory and/or pace of the airship.

According to another aspect, the present invention provides a method of detecting moisture content and particularly soil moisture content, as defined in claim 6.

According to the invention neutrons are at least partly moderated by means of the carrier gas. According to yet another embodiment, the neutron detector may be a gas proportional detector, particularly a boron neutron converter and more particularly a Jalousie detector and/or a scintillation neutron detector and/or a semiconductor neutron detector. According to the invention, the at least one neutron detector is positioned inside a carrier gas filled volume of the airship.

According to yet another embodiment, the method may comprise:
- detecting at least one out of humidity, pressure, background radiation, background neutron density, floating altitude, temperature, time lapse, weight of a carried object, carrier gas volume and/or carrier gas pressure, geomorphology and/or natural vegetal cover of the below area; and/or
- imaging the geographic landscape.

According to yet another embodiment, the method may comprise:
- controlling dynamically and/or in a predetermined manner conditions and/or features and/or functions of the apparatus, such as floating velocity, position, floating altitude, carrier gas pressure and/or detection of measures by means of at least one control element out of a remote control, a computer, a smartphone, a satellite, or a base station; and/or
- exchanging data between the control element and the apparatus.

According to yet another embodiment, the method may comprise:
- floating in an altitude between about 5 and about 200 m above ground; and
- screening the observation area of the earth's surface by means of floating in predetermined floating directions; or
- positioning the airship at fixed geographic coordinates; and/or
- fixing the airship to at least one reference point on the ground by at least one physical means, particularly a rope.

A preferential altitude between 5 and 200 m allows screening a large area compared to areas probed with conventional techniques. Floating however at a low altitude - around 5 to 10 and up to 50 m - allows probing the soil moisture content locally with higher precision and improved spatial resolution. If larger areas are to be screened, possibly in rough terrain and/or above a city or a forest, the airship may float at higher altitudes, for example at altitudes of 100 to 300 m. Depending on the requirements, the airship can float at different altitudes. Therefore, the method of detecting soil moisture content in the said altitude range allows versatile applications for different requirements or limitations due to the landscape or the operator's desires.

A dynamic control of the airship's position is favorable if an operator does not want to predetermine a trajectory. However, pre-setting a trajectory of the airship may be beneficial as automated aviation requires less man power and a remote control/computer autonomously controls the position and the pace of the airship. This may include that the airship can be operated as a drone.

The airship may be fixed to a position with respect to the geographic coordinate if the development/change of a condition at a certain position/small area (instead of a large area) over a longer time span is required to be probed. A fixed position of the airship can be achieved by dynamically controlling and possibly correcting for drift due to wind or by tethering the airship by means of at least one rope to a fixed position on the ground. The rope may not only be used to fix the airship but also to move it in a controlled manner to a new position, if required. In such cases, the airship may also be a balloon. However, it may be beneficial in terms of versatility to have the option of either fixing the airship to a certain position or moving it over the preferred or by predetermined landscape using either a rope and/or a remote control operated by an operator and/or a machine.

As the moisture measurement apparatus can be cost-effective, reliable, versatile, efficient, and handled in a straightforward manner, it may particularly be used in rough terrain, areas which often suffer ecological disasters and particular less developed countries which suffer poverty and a low degree of infrastructure. Such countries often experience ecological disasters related to flooding and landslip causing death of humans, animal stock and/or loss of property. Further, the moisture measurement apparatus may be applied by farmers to estimate the soil moisture content and to derive efficient/optimal amounts of irrigation. Again, less developed countries, often suffering water scarcity, may benefit in particular from the moisture measurement apparatus. The moisture measurement apparatus may therefore be configured to be used by either a private person, such as a farmer or a professional operator, such as a meteorologist or a person on behalf of the government or a company. The training for becoming an operator of the moisture measurement apparatus may therefore be simple depending on the requirements.

Further embodiments, aspects/features, advantages and functions are described in more detail as follows. It is self-evident, that features which do not contradict each other can be assumed combinable and therefore the below examples are not restricted to particular embodiments.
- **Fig. 1**: is a schematic drawing for demonstrating the function of the moisture measurement apparatus;
- **Fig. 2**: is a detailed schematic drawing of the moisture measurement apparatus;
- **Fig. 3**: is a diagram showing an energy distribution for neutrons measured in the atmosphere over soil with different soil moisture content;
- **Fig. 4**: is a diagram showing the radial sensitivity of neutrons detected at four different altitudes over soil with either high or low soil moisture content;
- **Fig. 5**: is a cut/cross section between the lines q and q' of the moisture measurement apparatus of Figure 2.

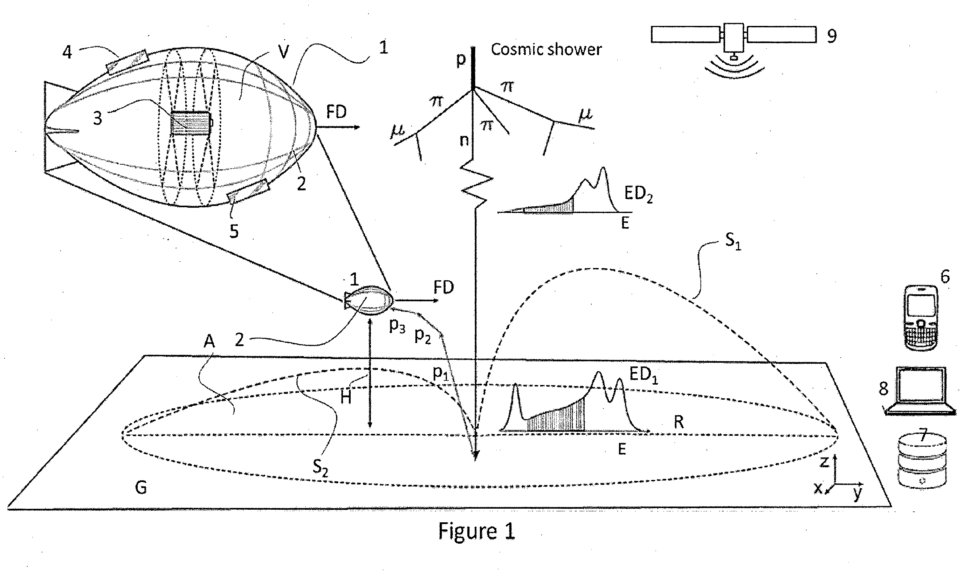

**Fig. 1****,** **Fig. 2** and **Fig. 5** show a spatial three dimensional coordinate system, wherein the orthogonal directions are indicated by arrows related to the reference signs x, y, z. In this representation x and y refer to a horizontal plane or a surface at one particular altitude, i.e. for example a flat ground G, neglecting the earth's curvature in an approximation. z on the other hand, indicates the vertical direction. The arrow for the vertical direction z is directed "upwards", i.e. towards higher altitudes. In other words, the arrow is directed from the ground G of the earth towards the sky/top of the atmosphere.

**Fig. 1** schematically illustrates one embodiment of the invention, also containing features which are not essential for the invention. Such additional features may be indicated as being optional. In other words, some aspects - particularly the aspects related the dependent claims - as illustrated in the figures may not limit the scope of the invention, as such aspects are not required for realizing the invention according to the independent claims.

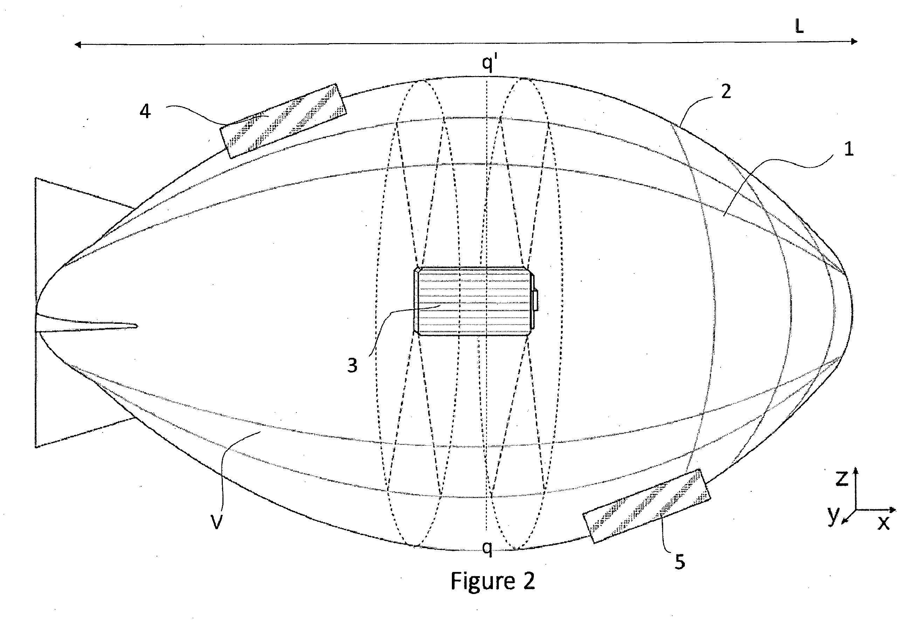

Referring to **Fig. 1** and **Fig. 2****,** an apparatus 1 for probing soil moisture content by means of detecting neutrons is described in the following. It is self-evident that a method for probing soil moisture content by means of detecting neutrons is covered by the same description. When referring to neutrons, mostly cosmic ray induced atmospheric neutrons are regarded. However, also cosmic neutrons may be detected and should therefore be covered by the term. The moisture measurement apparatus 1 according to the present invention is configured to detect moisture content, preferably soil moisture content. Alternatively or additionally, it may be possible to also detect moisture content related to biological cover (grass, trees, bushes or forest), fog, mist, clouds, or snow fields.

The moisture measurement apparatus 1 according to the present invention comprises at least one airship 2 and at least one neutron detector 3, which is positioned inside the airship as indicated in **Fig.** 1 and Fig. 2. The neutron detector 3 is configured to detect particularly thermal neutrons. These thermal neutrons may result from slowing down faster neutrons to energies which can be efficiently detected by the neutron detector 3, as will be described in more detail below. The detector 3 can either be a gaseous hybrid detector with solid neutron converters like ¹⁰B or a gaseous detector with ³He for example. Preferably, the detector 3 comprises several layers of boron carbide coatings on a cathode, Argon-CO₂ as a counting gas and a wire based anode, which is based on the principle of a proportional tube or a multi wire proportional chamber.

In more detail, the neutron detector 3 is described with respect to **Fig. 1** and **Fig. 2** which illustrate the apparatus 1 comprising one neutron detector 3. Alternatively, particularly for long term measurements a plurality of neutron detectors 3 may be provided. Preferably, the neutron detector 3 may comprise a gas proportional detector. More preferably, the neutron detector 3 may comprise a boron (¹⁰B) neutron converter, particularly a Jalousie detector. As opposed to ³He-detectors, which are often used for neutron detection, as they are compact and efficient, the boron neutron detectors are less expensive. ³He is so expensive (1500 $/l) that only small amounts/volumes can be provided for use in neutron detectors 3 such that only long probing intervals limited by the count rate (typically 1 hour) are affordable. ^{1G}B-solid state detectors are much cheaper. Therefore, larger detection volumes can be used resulting in higher neutron count rates and short probing intervals (typically a few minutes) can be realized for a typical accuracy which scales with the square root of the counted neutrons. In other words, using a preferred ¹⁰B-based neutron detector such as a Jalousie detector, a large area may be scanned, as the time required for the measurement at a single point at the surface of the landscape may be much shorter as opposed to the case in which ³He-detectors are used. Further, if the airship 2 floats at very slow pace, a high spatial resolution and/or a high degree of reliability may be achieved as the time for averaging the measured data over a certain area is long. The preferred ¹⁰B detector comprises a wire chamber and boron carbide converters being specifically designed to fit the geometry of the airship 2, which is a blimp in **Fig. 1** and **Fig. 2****.** Preferably the neutron detector 3 is a ¹⁰B-based detector and more preferably a so-called Jalousie detector.

However, it may also be possible in alternative embodiments that a ³He-detector is provided alone or in addition to some other neutron detector(s). The neutron detector 3 may also comprise a scintillation neutron detector and/or a semiconductor neutron detector as an alternative or in addition to other neutron detector(s).

In any case, it is preferred to apply a neutron detector which is specifically (or most) sensitive to thermal neutrons, i.e. neutron with a kinetic energy of about 25 meV, or an energy in the range of up to about 0.05 eV. Such neutron detectors are widely available and relatively easy to handle and to maintain. Moreover, when using such a detector in combination with a moderator according to the present disclosure, it turned out that a neutron density of thermal neutrons detected by the detector can be used as a reliable measure of soil moisture content, as will be described in more detail further below.

As already mentioned, the moisture measurement apparatus 1 comprises at least one airship 2, preferably the apparatus 1 comprises one airship 2 as illustrated in **Fig. 1****.** The airship 2 may also be known as or comprise a dirigible or aerostat. The term airship 2 may comprise non-rigid (e.g. blimps, balloons such as tethered or moored balloons, and free-floating balloons etc.) or semi-rigid airships. Even though the airship 2 may also be a rigid airship (e.g. Zeppelins), it is preferred that the moisture measurement apparatus 1 comprises an airship 2 having a non-rigid structure. An airship 2 with a non-rigid structure can be folded and packed when its volume V is not yet filled with gas, e.g. before operation.

The weight of an airship 2 is mainly due to its hull. However, the weight may also be substantially determined by the weight of the fuel, which may be high for long distance flights and/or windy conditions. It may be preferential to choose a heavier airship 2 if operating under rough conditions, such as strong winds, as a heavier airship 2 tends to be more stable against changes of wind directions. The overall weight of an according measurement apparatus 1 may be up to 1000 kg. Preferably, the weight of the measurement apparatus 1 comprising the airship 2 is however low, particularly around 100 kg. Therefore, that transportation can be simplified. In other words such an airship 2 according to the preferred embodiment has a low weight and can adopt a very small volume V when it is not in an operational mode, for example when it is sold or sent to a customer or transported to the preferred destination where it should be operated.

Preferably, the airship 2 is a blimp and adopts a length L between 1 and 20 m, when in the operating configuration, i.e. when being filled with the carrier gas and ready to float. More preferably, the length L of the airship 2 is approximately 10 m. Preferably, the airship 2 is configured to be remote controlled by an operator and/or a machine via wireless connection to a remote control 6 and/or computer 8 and/or other control element. Preferably, the airship 2 is configured to float in a floating direction FD in order to scan and probe a certain area on the ground G. In the preferred embodiment the airship 2 is configured to be remote controlled via wireless connection. In another preferred embodiment the airship 2 is configured to be remote controlled via wireless connection and by means of a rope. The airship 2 may float using an engine which is positioned at the airship as illustrated in **Fig. 1****.**

Alternatively or additionally, the airship 2 may be driven externally via a rope which is either guided by an operator by hand and/or by an engine on the ground G. The airship 2 may also be tethered to a fixed position on the ground G. Preferably the airship 2 is configured to float in a floating direction FD and to be located at a fixed position, e.g. by means of a rope which tethers the airship 2 to the ground G.

Alternatively, the airship may also have a rigid structure, may also be heavier than 100 or even heavier than 1000 kg and/or may not be foldable into a compact shape. Therefore, the airship 2 may adopt a length L either smaller than 1 meter, for example if the airship 2 comprises a balloon, or longer than 20 meters if the airship 2 comprises a Zeppelin. It may be necessary in an optional embodiment that the volume V is at least partially maintained particularly if the airship 2 has a semi-rigid or rigid structure. However, such an airship 2 in a non-operational state may be filled with a non-carrier gas such as air.

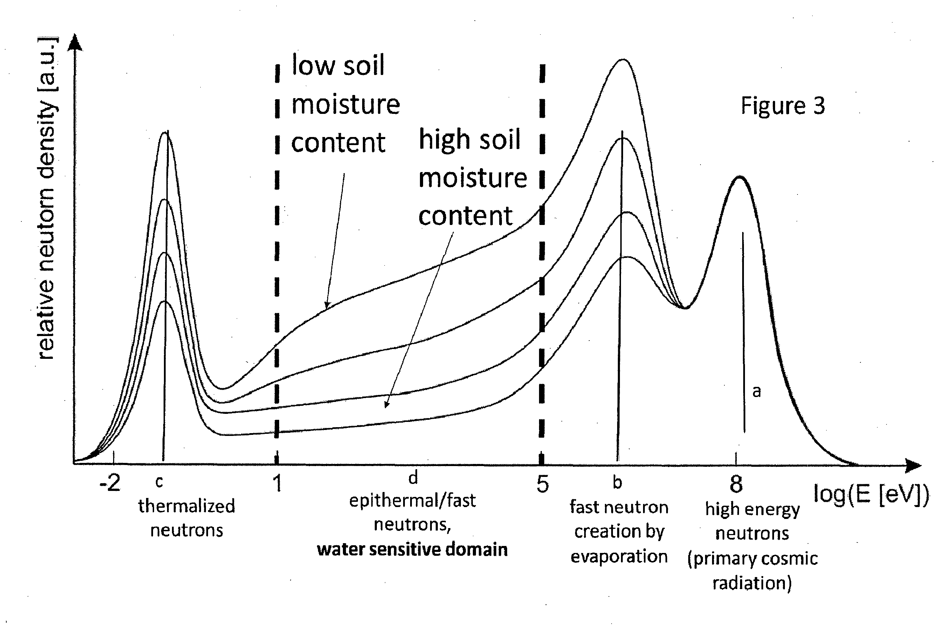

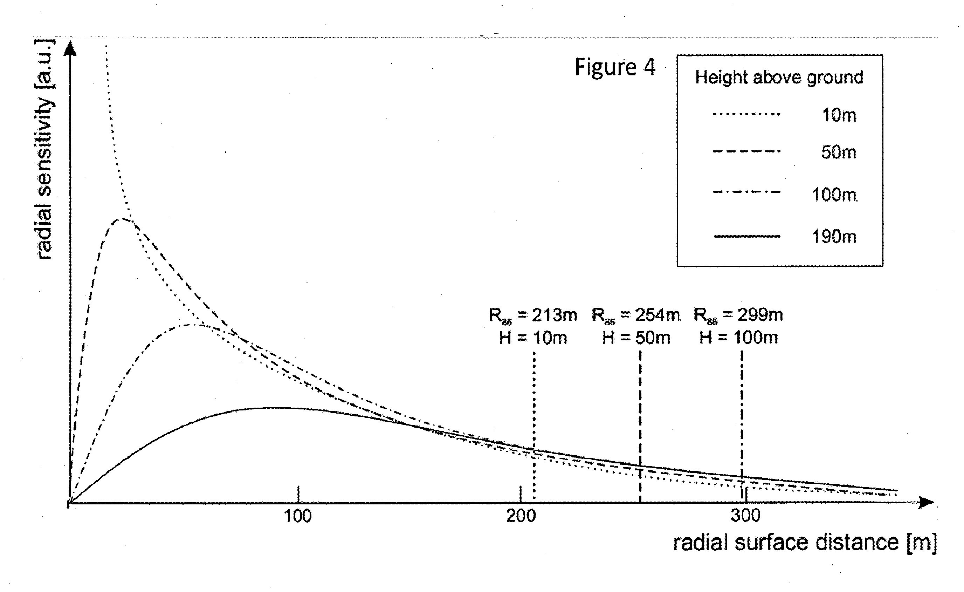

Preferably, the airship 2 floats at altitudes H between about 5 m and about 300 m, more preferably between about 20 m and about 200 m and even more preferably between about 50 m and about 150 m. The upper limit of the altitude H is thereby mainly limited by the average free path length of neutrons being reflected from the soil, wherein the average free path length is related to the signal strength, i.e. a measure related to the neutron density/count rate. The average free path length is a measure for the average distance for a particle needed to interact with another particle. In **Fig. 1****,** the average free path length is schematically illustrated using the reference signs p₁, p₂, p₃. As schematically indicated by the length of the arrows, the average free path length decreases with rising altitude H, as the number of scatter events rises causing a loss of neutron energy. This process is described below in more detail with respect to **Fig. 3** and particularly **Fig. 4****.** It is hence required to adjust or optimize the altitude H for a large enough probing area A and a strong enough neutron counting signal in view of the said trade-off. Alternatively, the airship 2 may be configured to float at altitudes H higher than about 300 m.

Preferably, the airship 2 floats at a slow pace over ground G between about 0 and about 50 km/h, even more preferably between about 0 and about 20 km/h. The time/period required for detection of one measurement point (typically in the range of minutes) and of a typical favorable area (typically several hectares) at a high enough spatial resolution and reliability corresponds well to the said floating pace. Alternatively, motors may be provided which allow a large airship 2 to float at a pace up to about 125 km/h. However, light airships typically do not exceed a pace of 50 km/h. Floating at such high pace or even faster is however not preferred, as the measurements suffer loss in reliability and spatial resolution. Therefore, it is favorable and advantageous to provide an airship which is able to float at very slow pace as opposed to other aircrafts, such as gyrocopters, helicopters or small air planes. Preferably, the pace is derived from the preferred duration needed for the measurement, the size of the area to be scanned, the preferred spatial resolution and the desired reliability to be achieved.

At least parts of the volume V of the airship 2 - when in an operating state and floating or ready for floating - are filled with a carrier gas. Preferably, the carrier gas contains elemental or molecular hydrogen atoms and more preferably the carrier gas comprises hydrogen and/or methane. Alternatively, the carrier gas may be one or more out of other gases which are lighter than air, such as helium, heated air, ammonia, neon, acetylene, diborane, carbon monoxide, nitrogen and ethene.

The carrier gas which is provided in the volume V of the airship 2 also acts at least in parts as a moderator medium for slowing down neutrons. It is required to slow down fast neutrons, as the cross section for the conversion reaction, required to detect neutrons, increases inverse-proportionally with respect to the decreasing speed of the neutron. To efficiently detect neutrons, a high detection rate (reactivity between neutrons and detector material of the neutron detector 3) is necessary, which calls for slow neutrons. Therefore efficiently moderating the neutrons is required to enable a high detection rate. It is preferred to at least partially provide elemental or molecular hydrogen atoms as moderator, as the hydrogen nucleus, the proton, is most efficient in moderating neutrons due to its equal/similar mass. Most preferably, in view of efficiency, hydrogen is used as a moderator medium - also with respect to the carrying properties and the prize. Another preferable gas to be used as a carrier medium and a moderator medium is methane.

Alternatively, heated air (possibly with water content or hydrogen content) may also be used at least as a carrier gas and possibly as a moderator medium. In another alternative, chambers are provided which separate gas portions inside the volume V of the airship 2 from each other. The neutron detector 3 is positioned in a chamber filled with a gas acting as a moderator medium and other chambers may comprise different gas which may have good carrying properties.

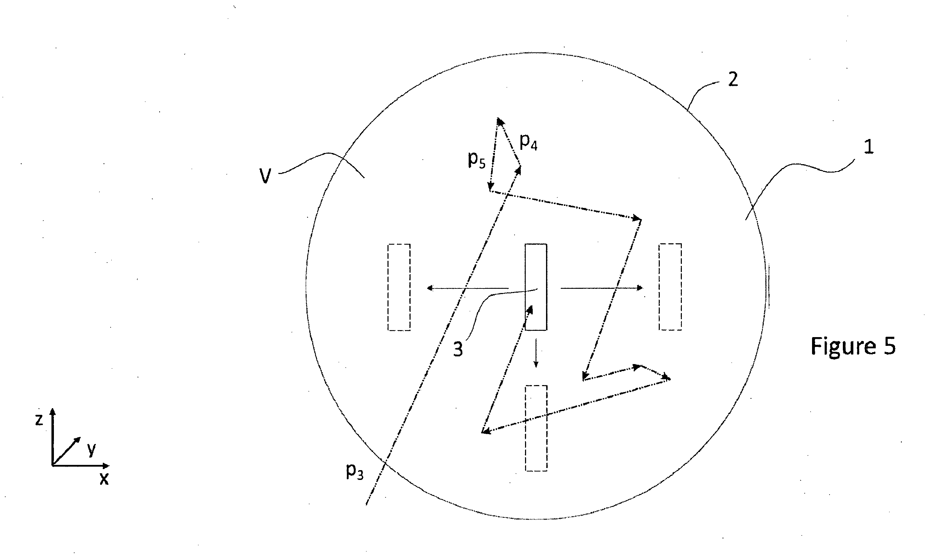

The neutron detector 3 is positioned inside the airship 2 such that the neutron detector 3 is surrounded by the gas acting as the moderator medium. As illustrated in more detail in **Fig. 2** and **Fig. 5****,** the neutron detector 3 is substantially positioned in the center of the volume V of the airship 2. In this preferable configuration, the neutron detector 3 is surrounded by the carrier gas.

Alternatively, the neutron detector 3 may also be positioned substantially in the bottom or in the upper, in the front or in the back portion of the volume V of the airship 2.

As indicated in **Fig. 1** and **Fig. 2****,** the moisture measurement apparatus 1 also comprises a secondary detector 5 to probe secondary measures which are not necessarily related directly to neutron sensing. Alternatively, more than one of such secondary detectors 5 may be provided at the airship 2, however according to the invention a secondary detector 5 is not explicitly required to realize the invention. As illustrated in **Fig. 1** and **Fig. 2****,** the secondary detector 5 is positioned on the outside of the airship 2. Alternatively, one or more secondary detectors 5 may also be positioned on the inside of the airship 2 or hanging down from the airship 2. One or more secondary detectors 5 may record measures such as humidity, barometric pressure, temperature, time and/or time lapse, background radiation, particularly background neutron density, floating pace and height/altitude H, position above ground G, weight of carried object, carrier gas volume/pressure, geomorphology and/or natural vegetation which covers the probed below area. Instead of or in addition to a secondary detector 5, a camera may be provided for imaging the geographical landscape such that measured values can be correlated to the particular position above ground at which the values have been detected. The camera may be a digital camera and/or a night-vision device.

The values/data obtained by means of secondary detector(s) and/or camera may serve to gain a more complete picture of the soil condition and/or the weather. For example, if a prediction or a warning system needs to be established, such values may be fed into a forecast model to derive and predict possible future conditions and probabilities. Further, the measured values may be of importance for the calibration of the neutron detection. For example, in order to isolate the signal/count rate related to soil water sensitive neutrons from the signal/count rate generated by the background radiation which is not sensitive to soil water content, a spectrum or signal/count rate over a local water source (e.g. a sea) may be recorded and subtracted from the spectrum or signal/count rate recorded over the observation area.

Further, barometric pressure and humidity may have an influence on the neutrons' energy, as more or less scatter events may take place before being detected. Such variations and sensitivities altering the measurement may be derived based on these values. By calibrating the measurement such effects can be excluded/subtracted/isolated from the measured signal to isolate the information which is indicative of soil moisture content only.

Background radiation and background neutron density may refer to radiation and neutron density, respectively, excluding the neutron density of neutrons which have been scattered by and/or interacted with soil water molecules. In other words, the signal/density value which is measured at the detector and which originates from background radiation and background neutrons does not originate from neutrons which are sensitive to soil water, as they have not been scattered from and interacted with soil water molecules. Therefore, such background data which are included in the measured spectrum are not indicative of soil water content and are therefore preferably subtracted from the measured spectrum or measurement rate/intensity.

As indicated in **Fig. 1** and **Fig. 2****,** the moisture measurement apparatus 1 also comprises a receiver and/or transmitter/transceiver 4 for communication with control elements (remote control and/or mobile phone 6, server 7, computer 8, and satellite 9) afar from the measurement apparatus 1. Alternatively, more than one of such transceivers 4 may be provided at the airship 2, however according to the invention a transceiver 4 is not explicitly required. As illustrated in **Fig. 1** and **Fig. 2****,** the transceiver 4 is positioned on the outside of the airship 2. Alternatively, one or more transceivers 4 may also be positioned on the inside of the airship 2 or hanging down from the airship 2. The transceiver 4 may be used to receive instructions which would for example be required if the airship 2 was remote controlled. For example, an operator or a machine at the ground G may - via remote control and/or mobile phone 6 and/or computer 8 - control the trajectory of the airship 2. This may be performed in a predetermined or dynamic manner. The transceiver 4 can also receive GPS signals from satellites 9 to determine the exact position with respect to the ground G. The position and/or floating pace may either be identified by a computer 8 on the ground G or by a computer in the GPS receiver, which may transmit the position to an operator or a machine on the ground G. It may also be possible that data are exchanged between transceiver 4 and control element on the ground G. For example, the data detected by the neutron detector 3 or the optional secondary detector(s) 5 may be instantly transmitted to a computer 8 and/or server 7 for storage and/or analysis. It may be a further option to transmit calibration data from a computer 8 to a transceiver 4 at the airship 2 for calibrating the detectors 3, 5.

For better understanding the technology of ray neutron sensing/probing, **Fig. 3****,** **Fig. 4** and **Fig. 5** are provided and referred to in the following discussion.

Radiation that is pounding the Earth originates mostly in our galaxy, e.g. from acceleration in shock regions of supernova remnants. Protons are the main part of the particle flux, accompanied by other charged nuclei. Cosmic rays penetrate the upper atmosphere and generate neutrons mostly by secondary collision processes. The spectrum is described in detail in **Fig. 3****.** At the top of the atmosphere it consists mainly of high energetic neutrons, which propagate towards the earth. As many neutrons get absorbed during this transport process the intensity of this incoming radiation decreases with atmospheric depth - or increases with altitude.

As indicated in the energy density distribution ED₂ related to high altitudes in **Fig. 1****,** a dominant peak is present in the energy density distribution (spectrum) at a high energy E. This peak is still visible in the energy density distribution ED₁ related to low altitudes in **Fig. 1****.** This indicates that cosmic neutrons also reach the ground G as their energy is extremely high and their average free path length is relatively long.

This peak at highest energy is centered between about 10 MeV and about 1 GeV, approximately at about 100 MeV, being characteristic for neutrons originating from a primary reaction with an extraterrestrial high-energetic particle at a fringe region/top of the atmosphere. Referring now to **Fig. 3****,** the energy density distribution of neutrons related to four different soil moisture contents is illustrated. The 4 different lines (from top to bottom) give an idea about the intensity distribution for 1%, 10%, 20% and 40% relative soil moisture. The rightmost peak indicates high energetic atmospheric and cosmic neutrons. This peak is characteristic for neutrons of primary reactions in the atmosphere by extrasolar particles indicated with the reference sign "a" and present in all curves in a high energy region. These neutrons make up a large part of the downward facing incoming radiation.

Neutrons may also be products/particles of secondary cosmic rays also known as secondary cosmic radiation (inset of **Fig. 1** referring to a possible reaction causing cosmic shower). Secondary cosmic-ray particles (e.g., muons, protons, pions, neutrons) are generated by electromagnetic and nuclear interactions mostly in the outer part of the Earth's atmosphere. In other words, such cosmic neutrons originate from nuclear reactions with particles at the fringe region of the atmosphere, the reactions caused by stellar or solar particles ("solar winds" for example), such as high-energetic protons as shown in the inset of **Fig. 1** or electrons and α-particles, when entering the atmosphere. The entity of particles which are generated in such events is therefore called particle/cosmic shower. In such nuclear reactions, high-energetic extraterrestrial particles hit atmospheric particles or particles at the fringe of the atmosphere and splitting of the particles may occur as a result, i.e. one particle is destroyed in favor of other particles which are generated in the event. Such a reaction in which (among others) neutrons are generated may take place as illustrated in the inset of **Fig. 1****.** As illustrated therein, a high energetic stellar or solar proton collides with an atom or molecule at the fringe of the atmosphere. Typically, high-energetic protons induce spallation of nitrogen or oxygen nuclei in the atmosphere. A variety of particles, such as µ, π, ν, γ, electrons and neutrons are generated either in the reaction or in a cascade of reactions. These products, called secondary particles/radiation, may also be high-energetic causing further cascade reactions.

Besides nuclear reactions, events also comprise elastic scattering. A neutron may for example lose energy by colliding and elastic scattering with atmospheric light particles, particularly hydrogen atoms having similar mass. The more scattering events a neutron has experienced, the less energy it has. This correlates directly with the so-called average free path, a measure for how far a neutron can travel before colliding with a particle in average. Cosmic or atmospheric neutrons at very high altitudes did not yet experience many scatter events, still having a high energy and a long average free path length. In addition, as the pressure in high altitudes is very low due to a low atmospheric particle density, the probability for neutrons to collide with a particle is still low.

Referring to **Fig. 3****,** the energy regime for fast secondary neutrons is indicated by the reference sign "b". This second peak from the right (i.e. high energy side) is related to evaporation neutrons. These neutrons are released from interactions of high energetic cosmic particles with other non-hydrogen atoms. They have energies in the range of about one to several MeV. Referring to **Fig. 1****,** the energy distribution spectra ED₂, ED₁ for high and low altitudes each contain this peak.

Below roughly 1 MeV, neutrons are efficiently slowed down by elastic collisions, particularly in the range limited by the dashed bars as illustrated in **Fig. 3** (1 eV - 0.1 MeV). Thus, the soil acts as a reflector and generator or moderator of neutrons. The process of moderation (slowing down) of fast neutrons is most efficient for collisions with hydrogen. Over a wet body (e.g. wet soil or a water surface such as lake) the slowing down is more efficient needing less collisions to lose energy.

All other typical elements are significantly heavier than hydrogen, and therefore elastic collisions with such atoms do not primarily lead to a significant energy loss of scattered neutrons. Therefore, a wet soil efficiently moderates the incoming cosmic neutron radiation, while a dry soil rather reflects neutrons.

An efficient range in the spectrum for water-sensitive neutron detection preferably used in connection with the present disclosure is the energy range which is marked with the reference sign "d" and the two dashed bars as indicated in **Fig. 3****.** Particularly, the energy density distribution ED₁ curve for low altitudes of **Fig. 1** indicates a high neutron density of such epithermal neutrons near the ground G (the water sensitive energy range of the ED₁ curve being marked with multiple vertical bars). In regard to this energy range the number of neutrons, which carry an information of the soil condition, decreases with height as indicated by the energy density distribution ED₂ curve for high altitudes of **Fig. 1** (the water sensitive energy range of the ED₂ curve also being marked with multiple vertical bars). One example of a neutron path, which ends at the airship (e.g. blimp), is shown by the arrows (p₁, p₂, p₃) in **Fig. 1****.** The average free path length for epithermal neutrons is between about 50 m to about 200 m. Thus, this is the most preferred altitude above ground for using the present invention, i.e. most preferably the airship may float in altitudes in this regime, e.g. between about 10 m and about 300 meters.

The epithermal and fast regime extends downwards to the thermal regime. In average, neutrons are not slowed down any further as they have kinetic energies which correspond to the surrounding material and therefore the energy transfer by collisions on average is zero. Such neutrons scatter locally until they are absorbed. Typically neutrons are 'thermalized' by water after approximately 20 collisions. As such thermal neutrons have a significantly shorter path length and a higher absorption probability than epithermal neutrons, they are not transported over long distances. The peak indicated with reference sign "c" in **Fig. 3** corresponds to such thermal equilibrated neutrons. The same low energy peak is also present in the energy density distribution ED₁ for neutrons at low altitudes.

As visible from **Fig. 3****,** the peaks/regions related to fast, epithermal and slow/thermalized neutrons (b, d, c, respectively) reveal information about the soil moisture content. Due to the sensitivity to moisture content, the energy range related to fast and epithermal neutrons is preferably selected for deriving soil moisture content, particularly the region which is indicated by the two dashed bars in **Fig. 3****.**

The peak, related to high energy neutrons does not reveal a sensitivity to soil moisture content as the neutrons' energy is too high and therefore the neutrons' cross section for interaction with water molecules is too small.

The neutrons of particular interest are slowed down to energies of thermal neutrons which can be efficiently detected by the detector 3. Thermal neutrons are free neutrons having a kinetic energy distribution with a peak at about 0.025 eV (i.e. about 4.0x10-21 J or 2.4 MJ/kg, corresponding to a speed of 2.2 km/s). This energy corresponds to the most probable velocity at a temperature of about 290 K, i.e. about 17 °C or about 62 °F (for the Maxwell-Boltzmann distribution for this temperature). After a number of collisions/scatter events with nuclei in a medium (e.g. a neutron moderator) at this temperature, initially epithermal neutrons arrive at about this energy level, provided that they are not absorbed. Referring to Fig. 3, the energy domain which characterizes thermal neutrons is the region of the spectrum indicated by "thermalized neutrons".

The effective neutron absorption cross-section of thermal neutrons for a given nuclide is different and often much larger than the absorption cross-section of fast neutrons. Therefore, such thermal neutrons can often be absorbed more easily by an atomic nucleus. In this process of absorption, a heavier, often unstable isotope of the chemical element is generated.

When approaching the moisture measurement apparatus 1, considerable portion of neutrons scattered and/or reflected at the observation area of the earth's surface is in the energy range of the characteristic energies for epithermal neutrons and/or fast neutrons, before being slowed down. The energy range of epithermal neutrons may be considered to cover a range between about 0.05 eV and about 0.4 eV. The energy range of fast neutrons may be considered to cover a range between about 1 MeV and about 20 MeV.

Neutrons which are particularly indicative of soil water content have energies which are in the water sensitive domain "d" of the spectrum. In this respect, it is referred to the gray square of **Fig. 3** as indicated by "epithermal/fast neutrons, water sensitive domain" or "d" (also indicated by the two dashed bars in **Fig. 3****).** Particularly the neutrons which are in the energy range between about 1 eV and about 100 keV are most sensitive to soil water content. The scattering cross-sections of the neutrons at such energies allow interaction such as scattering with nuclei in the soil, while absorption is comparatively small. Thus, the neutrons which are in this particular energy range when reaching the moderator before being slowed down/moderated by the moderator medium to energies of thermal neutrons are the neutrons of specific interest for the present soil water content measurement. It is pronounced, that the neutrons of interest for soil water content determination do have energies in the water sensitive domain (i.e. 1eV to 100 keV) when reaching the moderator and before interacting with the moderator medium. After the process of moderation, the neutrons have energies which are characteristic for thermal neutrons. The detector 3 is efficiently able to detect such thermal neutrons.

Neutrons which have energies outside of this water sensitive domain and which are therefore not sensitive to soil water molecules may be considered background neutrons. Such background neutrons may for example be fast or high-energy neutrons at energy ranges above 100 keV. In this respect, it is referred to the energy domains indicated by "fast neutron creation by evaporation" and "high energy neutrons (primary cosmic radiation)". Also neutrons which are already thermalized when reaching the airship 2 and which are not sensitive to soil water content are considered background neutrons. The background neutrons do not contribute to the determination of soil water content. Therefore the part of the neutron density/energy spectrum generated by background neutrons is required to be subtracted from the measured spectrum in order to extract the part of the spectrum from which the water content can be derived.

As mentioned before, the neutrons in the epithermal energy range are highly sensitive to soil moisture content. The neutron detector 3 is specifically configured to detect neutrons which are in this energy range when they approach the airship 2. However, to increase the probability of being detected related to the reaction cross section, those epithermal neutrons are further slowed down/moderated right before entering the detector. As preferably ¹⁰B-neutron detectors such as Jalousie detectors are used for the present invention, the reaction/interaction between ¹⁰B and neutron follows most likely the following conversion reaction:

¹⁰B + n → ⁷Li* + α → ⁷Li + α + γ + 2.31 MeV.

The detector, which is preferably a proportional counter, in fact measures the charges which are generated proportionally with the number of reactions caused by the neutrons. A processing unit of a computer 8 may then analyze the measurement and derive a neutron density. As one result an angular dependence of the neutron density is indicative of the soil moisture content, as illustrated as the dashed lines (S₁, S₂) of **Fig. 1****.** The dashed lines therefore show the radial sensitivity of neutrons having epithermal energy. The neutron density is measured from the center of the area A at the floating altitude H of the sensor. Here the cases of dry (right dashed line S₁) and wet soil (left dashed line S₂) are illustrated as radial intensity distributions. In other words, **Fig. 1** illustrates schematically the angle and moisture content dependencies of the neutron density in the signal curves S₁, S₂. The less moisture is contained in the soil - as indicated by signal curve S₁ - the more neutrons are "reflected" back from the surface of the earth as the neutrons interact with a smaller number of water molecules which cause scattering. For the case in which the soil contains high moisture content - as indicated by curve S₂ - less neutrons are reflected from the earth's surface towards the airship as more scattering between water molecules and neutrons takes place.

The neutron energy is an indirectly measured quantity. The neutron counter counts thermal neutrons. The moderator slows down neutrons to thermal energies, which is a statistical process. Thus, the kinetic energy before entering the device (1) can only be estimated by means of ensemble statistics.

In **Fig. 4****,** a more detailed diagram illustrates the dependence between the detected neutron density/radial sensitivity (y-axis) and radial surface distance from the detector (x-axis) and floating altitude/height above ground (dotted, dashed, dash-dotted, solid lines). Curves for four different altitudes, i.e. 10, 50, 100 and 190 m above ground G are plotted (dotted, dashed, dash-dotted and solid, respectively), against the radial surface distance R from the detector 3, as already indicated in **Fig. 1** with reference sign "R". All curves refer to a soil moisture content of 10 %.

As can be seen from the curves related to the altitudes H of 50 m (dashed lines), 100 m (dash dotted lines) and 190 m (solid lines), each curve peaks at a certain radial distance R. The according maxima occur at short distances R for low altitudes H. For example, the curves related to a floating altitude H of 50 m (dashed lines) reveal a maximum neutron density for a radial distance R between source and position over ground G of the airship of approximately 25 m as opposed to 80-90 m for the curves related to the altitude H of 190 m. The maxima of the curves related to an altitude H of 10 m is not visible in the present diagram as the value is too large to be in the same diagram with the other curves. It is also obvious from the diagram that the curves' shapes are flattened out for higher altitudes H, which is due to the higher number of scattering events experienced by the neutrons which are on the way from the ground G to the airship 2. The more neutrons experience scattering, the more energy is lost per neutron, such that neutrons suffer loss in average free path length and equilibrate before reaching the airship 2 and the neutron detector 3. In other words, for high floating altitudes H, less neutrons reflected from the ground G arrive at the detector 3 as their energy and average free path lengths are low due to a high number of scattering events with molecules in on their paths in the air. When equilibrated, neutrons can be absorbed easily and their movement direction becomes random due to Brownian motion.

In **Fig. 4****,** radial surface distances R from the detector 3, at which 86 % of the overall neutron counting rate is measured, is illustrated as vertical lines for the first three altitudes H. The distance R is approximately 213 m (dotted vertical line) for an altitude H of 10 m. The distance R is 254 m (dashed vertical line) for an altitude H of 50 m and 299 m (dash-dotted vertical line) for an altitude H of 100 m. These values again reflect the above discussed dependencies of radial sensitivity and neutron detection rate being larger for lower altitudes H. Based on these dependencies and the detection requirements, such as spatial resolution, the size of the area to be scanned, duration required to complete a measurement for a certain area, it can be calculated what the optimum altitude H would be. If an operator does not desire to achieve a high spatial resolution but a large area needs to be scanned, it is advantageous to select an altitude H which is high, such as heights H between 150 and 300 m. If a small area A needs to be scanned at a high spatial resolution, it is required to select a low altitude H, such as 10 to 100 m.

For derivation of soil moisture content sensitivity, it may be required to determine the dependencies of other measures. Therefore, it may also be required to record or determine values which are essential for a calibration, such as humidity, temperature, floating altitude H and neutron density measured over moisture-saturated ground which can be performed by detecting the neutron density over a lake. The values which may be required for calibration, such as floating altitude H, temperature, position, neutron density measured over moisture saturated ground or others may be detected at the airship 2. Therefore, one or more detectors for secondary measurements may be installed at the airship 2. Other values, which may be required for calibration such as solar activity and related intensity of cosmic neutron generation may be achieved from published sources, such as neutron monitor stations online. Such values may be transmitted for calibration to an optional machine carried by the airship 2. The calibration may be automated and performed by a machine or it may be done "by hand". The values may be entered into a program automatically or the data may be entered into a program "by hand". The calibration program may be stored on a machine/computer which is carried by the airship or the calibration program may be stored on a machine/computer which is on the ground, possibly implemented in a base station. The secondary data which might be measured at the airship may be transmitted to a machine and/or remote control on the ground for calibration and/or recording.

**Fig. 5** is a cut/cross section of the moisture measurement apparatus 1 between the points q and q', as indicated in **Fig. 2****.** The drawing schematically illustrates how a neutron on its path is slowed down by the moderator in the moderator gas-filled volume V of the airship 2. Initially the neutron of interest has an average free path length p₃ (see also **Fig. 1****)** when approaching and entering the volume V of the airship 2 related to an energy, which is in the range of epithermal neutrons (as already discussed in relation with **Fig. 3****).** By colliding with the moderator particles, which are also used as the carrier gas in the airship 2, the neutrons are slowed down to lower energies. Therefore the probability for the above described interaction with the detector medium becomes high as the effective cross section for an interaction increases with lower energies. In the above case for the above described conversion reaction, the detector medium is ¹⁰B, related to a preferred embodiment. In line with the energy loss, the average free path length - as indicated as an example by the length of the arrows of p₄ and p₅ - becomes shorter before entering the neutron detector 3.

The position of the detector 3 as indicated as a solid box in **Fig. 5** is preferably centered with respect to the volume V of the airship 2. Alternatively, the detector 3 may have a position which is different from the center, as indicated by the dashed boxes. Depending on the position, the detector 3 may then be more selective to neutrons with a certain incoming direction, as the neutrons coming in from one side may not be slowed down to the same extent as the neutrons coming in from another side.

### List of Reference Numerals

- 1: moisture measurement apparatus
- 2: airship
- 3: neutron detector
- 4: transceiver
- 5: detector for secondary measures
- 6: remote control / mobile phone
- 7: server
- 8: computing device
- 9: satellite
- a: peak energy
- A: area of the earth's surface (observation area)
- b: peak energy
- c: peak energy
- d: energy region for epithermal/fast neutrons
- E: energy
- ED₂: energy distribution of neutrons in high altitudes / in the upper atmosphere
- ED₁: energy distribution of neutrons in low altitudes / near the Earth's surface
- FD: floating direction
- G: ground
- H: floating height / altitude
- L: length of the airship
- p₁₋₅: average free path length of a cosmic ray induced neutron (CRAN)
- q, q': points between cutting line
- R: radius of a circular area for a certain signal intensity cut-off
- S₁: angle dependence of the neutron density measured at low soil moisture content
- S₂: angle dependence of the neutron density measured at high soil moisture content
- V: gas-filled volume of the airship
- x: spatial coordinate
- y: spatial coordinate
- z: spatial coordinate

## Claims

1. Moisture measurement apparatus (1) for detecting moisture content, particularly soil moisture content, the apparatus (1) comprising:
- an airship (2) configured to float over an observation area (A) of the earth's surface, when a carrier gas filled volume (V) of the airship (2) is at least partly filled with a carrier gas;
- a neutron moderator carried by the airship and configured to slow down neutrons scattered and/or reflected at the observation area of the earth's surface, wherein the carrier gas is configured to at least partially serve as the neutron moderator;
- a neutron detector (3) at least partly surrounded by the neutron moderator and configured to detect a neutron density, wherein the at least one neutron detector (3) is positioned inside the carrier gas filled volume (V) of the airship (2); and
- an evaluation module configured to determine the moisture content from the detected neutron density.

2. Moisture measurement apparatus (1) of claim 1, wherein the carrier gas is at least partially one out of hydrogen, helium, heated air, methane, ammonia, neon, acetylene, diborane, carbon monoxide, nitrogen and ethene or a mixture thereof.

3. Moisture measurement apparatus (1) of any one of the preceding claims, wherein the neutron detector (3) comprises at least one gas proportional detector, particularly a boron neutron converter and more particularly a Jalousie detector and/or a scintillation neutron detector and/or a semiconductor neutron detector.

4. Moisture measurement apparatus (1) of any one of the preceding claims, wherein the apparatus (1) further comprises at least one:
- second measurement device configured to detect at least one out of humidity, pressure, background radiation, background neutron density, floating altitude (H), temperature, time lapse, weight of a carried object, carrier gas volume and/or carrier gas pressure, geomorphology and/or natural vegetal cover within the observation area (A) of the earth's surface; and/or
- camera configured for imaging a geographic landscape.

5. Moisture measurement system comprising:
- at least one control element out of a remote control (6), a computer (8), a smartphone (6), a satellite (9), or a base station; and
- a moisture measurement apparatus (1) of any one of the preceding claims, configured to exchange data with the at least one control element, wherein data may be exchanged between the control element and the moisture measurement apparatus (1) for dynamical control over and/or predetermination of conditions and/or features and/or functions of the moisture measurement apparatus (1), such as float velocity, position, floating altitude (H), carrier gas pressure and/or detection of measures are controlled dynamically and/or in a predetermined manner.

6. Method of detecting moisture content and particularly soil moisture content comprising the steps of:
- providing at least one airship (2) having a carrier gas filled volume (V) at least partially filled with a carrier gas, the airship floating over an observation area (A) of the earth's surface;
- carrying at least one neutron detector (3) with the at least one airship (2), such that the neutron detector is at least partly surrounded by a neutron moderator;
- detecting a neutron density by means of the neutron detector; and
- determining the moisture content from the detected neutron density, wherein neutrons are at least partly moderated by means of the carrier gas, wherein the at least one neutron detector (3) is positioned inside the carrier gas filled volume (V) of the airship (2).

7. Method of either one of claim 6, wherein the neutron detector is a gas proportional detector, particularly a boron neutron converter and more particularly a Jalousie detector and/or a scintillation neutron detector and/or a semiconductor neutron detector.

8. Method of claim 6 or 7, further comprising the step of:
- detecting at least one out of humidity, pressure, background radiation, background neutron density, floating altitude (H), temperature, time lapse, weight of a carried object, carrier gas volume and/or carrier gas pressure, geomorphology and/or natural vegetal cover of the below area; and/or
- imaging a geographic landscape.

9. Method of any one of claims 6 to 8, further comprising the step of:
- controlling dynamically and/or in a predetermined manner conditions and/or features and/or functions of the airship (2), such as floating velocity, position, floating altitude (H), carrier gas pressure and/or detection of measures by means of at least one control element out of a remote control (6), a computer (8), a smartphone (6), a satellite (9), or a base station; and/or
- exchanging data between the control element and the airship (2).

10. Method of any one of claims 6 to 9, wherein the method comprises:
- floating in an altitude between 5 and 200 m above ground (G); and
- screening the observation area (A) of the earth's surface by means of floating in predetermined floating directions (FD); or
- positioning the airship at fixed geographic coordinates; and/or
- fixing the airship (2) to at least one reference point on the ground (G) by at least one physical means, particularly a rope.

## Patentansprüche

1. Eine Feuchtigkeitsmessvorrichtung (1) zum Erfassen des Feuchtigkeitsgehalts, insbesondere des Bodenfeuchtigkeitsgehalts, wobei die Vorrichtung (1) Folgendes umfasst:
- ein Luftschiff (2), das konfiguriert ist, um über einem Beobachtungsbereich (A) der Erdoberfläche zu schweben, wenn ein mit Trägergas gefülltes Volumen (V) des Luftschiffs (2) zumindest teilweise mit einem Trägergas gefüllt ist;
- einen Neutronenmoderator, der vom Luftschiff getragen wird und konfiguriert ist, um Neutronen zu verlangsamen, die an der Beobachtungsfläche der Erdoberfläche gestreut und/oder reflektiert werden, wobei das Trägergas konfiguriert ist, um zumindest teilweise als Neutronenmoderator zu dienen;
- einen Neutronendetektor (3), der zumindest teilweise von dem Neutronenmoderator umgeben ist und konfiguriert ist, um eine Neutronendichte zu erfassen, wobei der mindestens eine Neutronendetektor (3) innerhalb des mit Trägergas gefüllten Volumens (V) des Luftschiffs (2) positioniert ist; und
- ein Auswertemodul, das konfiguriert ist, um den Feuchtigkeitsgehalt aus der erfassten Neutronendichte zu bestimmen.

2. Die Feuchtigkeitsmessvorrichtung (1) nach Anspruch 1, wobei das Trägergas teilweise aus mindestens einem der Folgenden besteht: Wasserstoff, Helium, erwärmte Luft, Methan, Ammoniak, Neon, Acetylen, Diboran, Kohlenmonoxid, Stickstoff und Ethen oder einem Gemisch davon.

3. Die Feuchtigkeitsmessvorrichtung (1) nach irgendeinem der vorstehenden Ansprüche, wobei der Neutronendetektor (3) mindestens einen gasproportionalen Detektor, insbesondere einen Bor-Neutronenkonverter und noch spezieller einen Jalousie Detektor und/oder einen Szintillationsdetektor für Neutronen und/oder einen Halbleiter-Neutronendetektor umfasst.

4. Die Feuchtigkeitsmessvorrichtung (1) nach irgendeinem der vorstehenden Ansprüche, wobei die Vorrichtung (1) ferner mindestens einen der Folgenden umfasst:
- eine zweite Messvorrichtung, die konfiguriert ist, um mindestens einen der Folgenden zu erfassen: Feuchtigkeit, Druck, Hintergrundstrahlung, Hintergrundneutronendichte, Schwebehöhe (H), Temperatur, Zeitspanne, Gewicht eines getragenen Gegenstandes, Trägergasvolumen und/oder Trägergasdruck, Geomorphologie und/oder natürlicher Vegetationsbewuchs innerhalb des Beobachtungsbereichs (A) der Erdoberfläche; und/oder
- eine Kamera, die konfiguriert ist zum Abbilden der geografischen Landschaft.

5. Ein Feuchtigkeitsmesssystem, das Folgendes umfasst:
- mindestens ein Steuerelement aus einer Fernbedienung (6), einem Computer (8), einem Smartphone (6), einem Satelliten (9) oder einer Basisstation; und
- eine Feuchtemessvorrichtung (1) nach irgendeinem der vorstehenden Ansprüche, die konfiguriert ist, um Daten mit dem mindestens einen Steuerelement auszutauschen, wobei Daten ausgetauscht werden können zwischen dem Steuerelement und der Feuchtemessvorrichtung (1) zur dynamischen Steuerung und/oder Vorbestimmung von Bedingungen und/oder Merkmalen und/oder Funktionen der Feuchtemessvorrichtung (1), sodass die Schwebe-Geschwindigkeit, -Position, -Höhe (H), der Trägergasdruck und/oder die Erfassung von Messungen dynamisch und/oder auf vorbestimmte Weise gesteuert werden.

6. Ein Verfahren zum Erfassen des Feuchtigkeitsgehalts und insbesondere des Bodenfeuchtigkeitsgehalts, das die folgenden Schritte umfasst:
- Bereitstellen von mindestens einem Luftschiff (2) mit einem mit Trägergas gefüllten Volumen (V), das mindestens teilweise mit einem Trägergas gefüllt ist, wobei das Luftschiff über einem Beobachtungsbereich (A) der Erdoberfläche schwebt;
- Tragen von mindestens einem Neutronendetektor (3) mit dem mindestens einen Luftschiff (2), sodass der Neutronendetektor zumindest teilweise von einem Neutronenmoderator umgeben ist;
- Erfassen einer Neutronendichte mittels des Neutronendetektors; und
- Bestimmen des Feuchtigkeitsgehalts aus der erfassten Neutronendichte, wobei Neutronen zumindest teilweise mittels des Trägergases moderiert werden, wobei der mindestens eine Neutronendetektor (3) innerhalb des mit Trägergas gefüllten Volumens (V) des Luftschiffes (2) positioniert ist.

7. Das Verfahren nach irgendeinem aus Anspruch 6, wobei der Neutronendetektor ein gasproportionaler Detektor ist, insbesondere ein Bor-Neutronenkonverter und noch spezieller ein Jalousie Detektor und/oder ein Szintillationsdetektor für Neutronen und/oder ein Halbleiter-Neutronendetektor.

8. Das Verfahren nach Anspruch 6 oder 7, das ferner den folgenden Schritt umfasst:
- Erfassen von mindestens einem der Folgenden: Feuchtigkeit, Druck, Hintergrundstrahlung, Hintergrundneutronendichte, Schwebehöhe (H), Temperatur, Zeitspanne, Gewicht eines getragenen Gegenstandes, Trägergasvolumen und/oder Trägergasdruck, Geomorphologie und/oder natürlicher Vegetationsbewuchs des darunterliegenden Bereichs; und/oder
- Abbilden einer geografischen Landschaft.

9. Das Verfahren nach irgendeinem der Ansprüche von 6 bis 8, das ferner den folgenden Schritt umfasst:
- dynamisches und/oder vorbestimmtes Steuern von Bedingungen und/oder Merkmalen und/oder Funktionen des Luftschiffes (2), wie z.B. Schwebegeschwindigkeit, -Position, -Höhe (H), Trägergasdruck und/oder Erfassen von Messungen durch mindestens ein Steuerelement aus einer Fernbedienung (6), einem Computer (8), einem Smartphone (6), einem Satelliten (9) oder einer Basisstation; und/oder
- Austauschen von Daten zwischen dem Steuerelement und dem Luftschiff (2).

10. Das Verfahren nach irgendeinem der Ansprüche von 6 bis 9, wobei das Verfahren Folgendes umfasst:
- Schweben in einer Höhe zwischen 5 und 200 m über dem Boden (G); und
- Untersuchen des Beobachtungsbereichs (A) der Erdoberfläche durch Schweben in vorgegebenen Schweberichtungen (FD); oder
- Positionieren des Luftschiffes an festen geographischen Koordinaten; und/oder
- Befestigen des Luftschiffes (2) an mindestens einem Referenzpunkt am Boden (G) mit mindestens einem physikalischen Mittel, insbesondere einem Seil.

## Revendications

1. Un appareil de mesure d'humidité (1) pour détecter la teneur en humidité, notamment la teneur en humidité du sol, l'appareil (1) comprenant :
- un dirigeable ou encore un aéronef (2) configuré pour flotter au-dessus d'une zone d'observation (A) de la surface terrestre, lorsqu'un volume rempli de gaz porteur (V) du dirigeable (2) est au moins partiellement rempli avec un gaz porteur ;
- un modérateur de neutrons porté par le dirigeable et configuré pour ralentir les neutrons dispersés et/ou réfléchis dans la zone d'observation de la surface terrestre, sachant que le gaz porteur est configuré pour servir au moins partiellement de modérateur de neutrons ;
- un détecteur de neutrons (3) au moins partiellement entouré par le modérateur de neutrons et configuré pour détecter une densité de neutrons, sachant que l'au moins un détecteur de neutrons (3) est positionné à l'intérieur du volume (V) du dirigeable (2) rempli de gaz porteur ; et
- un module d'évaluation configuré pour déterminer la teneur en humidité à partir de la densité de neutrons détectée.

2. L'appareil de mesure d'humidité (1) d'après la revendication 1, sachant que le gaz porteur est au moins partiellement de l'hydrogène, de l'hélium, de l'air chauffé, du méthane, de l'ammoniac, du néon, de l'acétylène, du diborane, du monoxyde de carbone, de l'azote et de l'éthène ou un de leurs mélanges.

3. L'appareil de mesure d'humidité (1) d'après l'une quelconque des revendications précédentes, sachant que le détecteur de neutrons (3) comprend au moins un détecteur proportionnel à gaz (*gas proportional detector*), en particulier un convertisseur de neutrons au bore et plus particulièrement un détecteur Jalousie et/ou un détecteur de neutrons à scintillation et/ou un détecteur de neutrons à semiconducteur.

4. L'appareil de mesure d'humidité (1) d'après l'une quelconque des revendications précédentes, sachant que l'appareil (1) comprend en outre au moins l'un des suivants :
- un deuxième dispositif de mesure configuré pour détecter au moins l'un des éléments suivants : humidité, pression, rayonnement de fond, densité neutronique de fond, altitude de flottement (H), température, laps de temps, poids d'un objet transporté, volume du gaz porteur et/ou pression du gaz porteur, géomorphologie et/ou couverture végétale naturelle dans la zone (A) de la surface terrestre observée, et/ou
- une caméra configurée pour l'imagerie d'un paysage géographique.

5. Un système de mesure d'humidité comprenant :
- au moins un élément de commande d'une télécommande (6), d'un ordinateur (8), d'un smartphone (6), d'un satellite (9) ou d'une station de base ; et
- un appareil de mesure d'humidité (1) d'après l'une quelconque des revendications précédentes, configuré pour échanger des données avec au moins un élément de commande, sachant que des données peuvent être échangées entre l'élément de commande et l'appareil de mesure d'humidité (1) pour commander dynamiquement et/ou prédéterminer des conditions et/ou caractéristiques et/ou fonctions de l'appareil de mesure d'humidité (1), de manière que la vitesse, la position, l'altitude de flottement (H), la pression des gaz porteurs et/ou la détection des mesures sont commandées de manière dynamique et/ou prédéterminée.

6. Un procédé de détection de la teneur en humidité et notamment de la teneur en humidité du sol, comprenant les étapes consistant à :
- fournir au moins un dirigeable ou encore un aéronef (2) ayant un volume (V) au moins partiellement rempli d'un gaz porteur, le dirigeable flottant au-dessus d'une zone d'observation (A) de la surface terrestre ;
- porter au moins un détecteur de neutrons (3) par au moins un dirigeable (2), de manière que le détecteur de neutrons soit entouré au moins partiellement par un modérateur de neutrons ;
- détecter une densité neutronique au moyen du détecteur de neutrons ; et à
- déterminer la teneur en humidité à partir de la densité neutronique détectée, sachant que les neutrons sont au moins partiellement modérés au moyen du gaz porteur, l'au moins un détecteur de neutrons (3) étant placé dans le volume (V) du dirigeable (2) rempli de gaz porteur.

7. Le procédé d'après l'une parmi la revendication 6, sachant que le détecteur de neutrons est un détecteur proportionnel à gaz, en particulier un convertisseur de neutrons au bore et plus particulièrement un détecteur Jalousie et/ou un détecteur de neutrons à scintillation et/ou un détecteur de neutrons à semiconducteur.

8. Le procédé d'après la revendication 6 ou 7, comprenant en outre l'étape consistant à :
- détecter au moins un parmi l'humidité, la pression, le rayonnement de fond, la densité neutronique de fond, l'altitude de flottement (H), la température, le laps de temps, le poids d'un objet transporté, le volume de gaz porteur et/ou la pression du gaz porteur, la géomorphologie et/ou la couverture végétale naturelle de la zone en dessous ; et/ou
- imager un paysage géographique.

9. Le procédé d'après l'une quelconque des revendications de 6 à 8, comprenant en outre l'étape consistant à :
- commander dynamiquement et/ou de manière prédéterminée des conditions et/ou des caractéristiques et/ou des fonctions du dirigeable (2), telles que la vitesse, la position, l'altitude de flottement (H), la pression du gaz porteur et/ou la détection de mesures au moyen d'au moins un élément de commande d'une télécommande (6), d'un ordinateur (8), d'un smartphone (6), d'un satellite (9) ou d'une station de base ; et/ou
- échanger des données entre l'élément de commande et le dirigeable (2).

10. Le procédé d'après l'une quelconque des revendications de 6 à 9, sachant que le procédé comprend le fait de :
- flotter à une altitude comprise entre 5 et 200 m au-dessus du sol (G) ; et de
- inspecter la zone d'observation (A) de la surface de la terre par le fait de flotter dans des directions de flottement prédéterminées (FD) ; ou de
- positionner le dirigeable à des coordonnées géographiques fixes ; et/ou
- fixer le dirigeable (2) à au moins un point de référence au sol (G) par au moins un moyen physique, notamment par une corde.
